# EUROPEAN PATENT SPECIFICATION

(11) **EP 0 523 180 B1**
(45) Date of publication and mention of the grant of the patent: **13.10.1993**
(21) Application number: 91908314.7
(22) Date of filing: 05.04.1991
(51) Int. Cl.: A61J 1/10

(54) **A BLOOD BAG FOR USE IN SEPARATING BLOOD COMPONENTS**
ZUM TRENNEN VON BLUTKOMPONENTEN DIENENDER BLUTSACK
SAC A SANG SERVANT A SEPARER LES COMPOSANTS DU SANG

(30) Priority: 06.04.1990 SE 9001271
(43) Date of publication of application: 20.01.1993
(73) Proprietor: OMEGA MEDICINTEKNIK AB, 115 29 Stockholm (SE)
(72) Inventor: UNGER, Peter, S-115 29 Stockholm (SE); WESTBERG, Eric, S-181 30 Lidingö (SE)
(74) Representative: Wiedemann, Bernd
(86) International application number: SE9100251
(87) International publication number: WO9115182

(56) References cited:
- EP-A- 0 420 757

## Description

The present invention relates to a blood bag for use in separating blood components, said bag having at least two connections from which pipes or hoses extend to side containers for the sterile transfer of separated blood components.

Blood bags are used generally in clinical blood-activities on a world-wide basis and comprise a flexible, collapsible container made of a relatively thin plastic material. When empty, the blood bag has an essentially rectangular or square shape and a relatively small extension in depth. The blood bags are normally produced from a thin-walled hose which is first cut and then sealed along an upper and a lower edge, or from so-called double foil, which is sealed around the bag so as to form an upper, a lower edge and two side edges. Hose connections and valve connections of different kinds can be incorporated in said edges when sealing the bag. By "one edge", as used in the following description, is meant one of these sealed edges.

It is known to use a system of three or four sterilized bags interconnected with hoses or pipes, when separating the components of blood. Blood is drained or tapped into one bag, which is then sealed, and the whole of the bag system is placed in a centrifuge and the blood is centrifuged, wherewith the blood separates into an upper plasma layer, and intermediate buffycoat layer and a lower layer of red blood cells. The bag system is then removed from the centrifuge and the separated phases in the blood bag are squeezed or pressed into the depending side-containers, either manually or with the aid of special presses.

A system of this kind is described in Swedish Patent Specification No. 7902761-1 (publ. No 416 378), according to which the blood is drained into a bag which has outlet openings in both the upper and the lower edges of the bag. When centrifugation is completed, the layer that contains blood plasma is pressed out through the upper outlet opening of the bag, whereas the layer that contains red blood cells is pressed out through the lower outlet opening of said bag. The intermediate buffycoat layer thus remains in the blood bag.

This manual handling of the centrifuged bags entails the risk of re-slurrying of the stratified layers, to a greater or lesser extent. Consequently, attempts have been made to automatize the process of pressing or squeezing the stratified layers into respective containers. In this connection, it is desirable that all bag connections are formed in one edge of the bag. This also facilitates rational manufacture of the integrated bag with the aid of existing blood-bag manufacturing machines.

An object of the present invention is to provide a blood bag for the separation of blood components and from which plasma and red blood cells can be pressed into the side containers while leaving the buffycoat fraction in said bag, and in which bag the side-container connections are arranged in one edge of the bag.

This object is achieved in accordance with the invention with a blood bag having the characteristic features defined in Claim 1.

Thus, the inventive blood bag has at least two connections from which pipes or hoses extend to side containers and in which connections are disposed in one edge of the bag. The first of these connections either communicates directly with the blood bag at said edge or is an openable connection, and the second of said connections communicates directly with the interior of said blood bag, or is an openable connection, at a given distance from said edge.

This enables a blood fraction present in the lower part of the bag to be pressed out through the upper edge of the bag. Plasma and red blood cells can thus be pressed out while leaving the buffycoat fraction in the bag, and the blood bags can also be used in general for purposes corresponding to the purposes of the blood bag described in Swedish Patent Specification No. 7902761-1 (publ. No 416 378).

A further advantage afforded by the inventive blood bag is that the lower fraction can be pressed from the bag while leaving the oldest and therapeutically least valuable red blood vessels in the bag, these cells settling at the absolute bottom of the bag.

The invention will now be described in more detail with reference to the accompanying drawings, in which
Figure 1 illustrates an embodiment of an inventive blood bag having two side containers connected thereto;
Figure 2 illustrates another embodiment of an inventive blood bag having side containers connected thereto;
Figure 3 is a longitudinal section view of one embodiment of a bag connection which includes a rod that can be pressed into the bag;
Figure 4 is a detailed view similar to Figure 3 and shows the rod pressed into the blood bag;
Figure 5 is a detailed view in longitudinal section of another embodiment of a connection comprising a rod-which can be pressed into the bag; and
Figure 6 is a view similar to Figure 5, showing the rod pressed into the bag.

Mutually similar components have been identified with the same reference signs in the various Figures of the drawings.

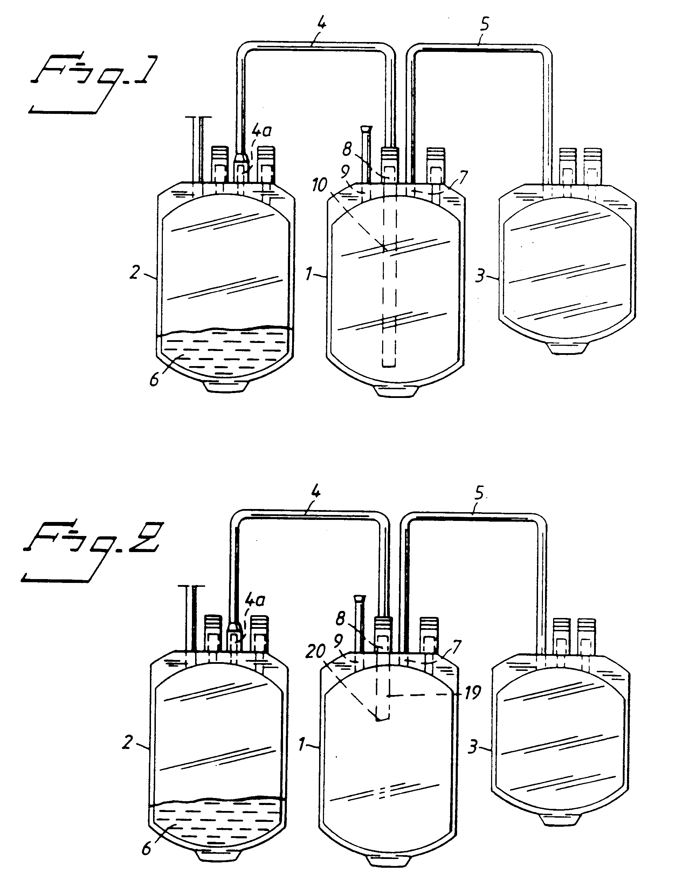

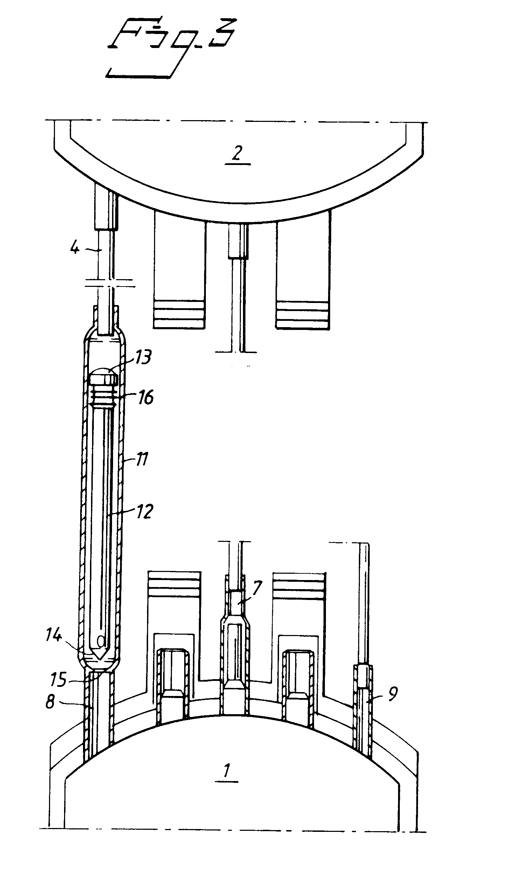

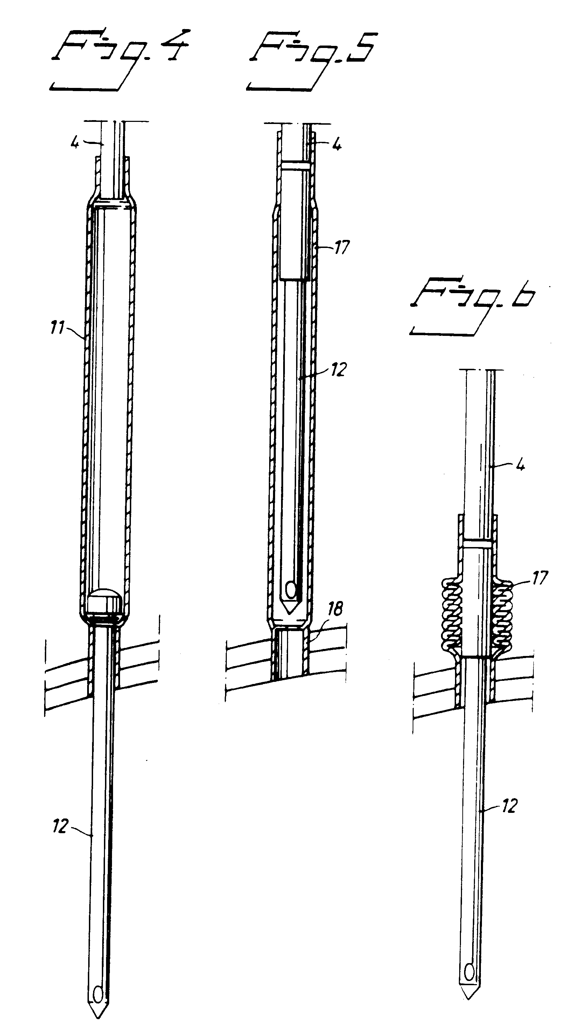

Figures 1 and 2 illustrate a set of mutually connected containers intended for use when dividing whole blood into plasma, buffycoat and erythrocyte cell fractions. The container set includes an inventive blood bag 1 which is provided with two connections 7, 8 in one edge of the bag. Connected to these connections 7, 8 are two side containers 2, 3, by means of conduits or hoses 4, 5, through which fluid is transferred from the blood bag to the side containers. Normally, at least one further connection 9 (Figure 3) is provided for the connection of a blood draining hose by means of which the bag is filled with the whole blood to be separated into its various components. This edge is normally positioned nearest the rotational center when centrifuging the bag and in the following is referred to as the bag top, whereas the opposite edge is referred to as the bag bottom.

The blood bag 1 may, for instance, be a standardized blood bag made of PVC-foil and intended to be filled with a standard blood unit of about 520 ml, including about 63 ml anticoagulant. The one side container 3 is intended for the collection of plasma and is connected to the top of the bag 1 by a conduit or hose 5, through the intermediary of a connector 7. The other side container 2 contains approximately 100 ml erythrocyte nutrient solution 6 (for example SAG-MAN) and is intended for collecting the erythrocyte cell concentrate or fraction. This second container is connected to the blood bag by means of a hose or conduit 4, which similar to the hose or conduit 5 is attached to a connector at the top of the bag.

In the embodiment illustrated in Figure 1, a conduit 10 is connected to the connector 8 and extends within the bag and terminates at a given distance above the bottom thereof. The conduit 10 may be a direct continuation of the hose 4 or may comprise a more rigid conduit or hose connected to the hose 4. Normally included in the circuit is a so-called shear pin 4a, i.e. a closure means which can be opened by breaking the shear pin. The conduit 10 is relatively long and terminates at the bottom of the bag, or a given distance above said bottom, such that the bottom orifice of the conduit will be located in the layer of erythrocyte cells subsequent to centrifugation. This embodiment enables plasma and cell concentrate to be pressed from the bag while leaving the intermediate buffycoat layer therein.

The embodiment illustrated in Figure 2 differs from the embodiment described with reference to Figure 1, primarily in that the conduit 19 extending within the bag is shorter than the conduit 10 of the Figure 1 embodiment, intended for a corresponding purpose. The length of the conduit 19 is such that said conduit will not reach the buffycoat layer formed in the centrifugation process, but opens into the plasma layer. When this blood bag is squeezed or compressed, the plasma is pressed out first. The buffycoat layer then rises in the bag so as to cover the orifice of the conduit 19 prior to opening the hose 4 and the erythrocyte cell concentration is pressed out into the side container 2. The advantage with this embodiment is that cell concentrate can be recovered free from contamination by the buffycoat fraction. The conduit 19 is filled with blood at the same time as the bag is filled. During subsequent centrifugation, however, buffycoat and erythrocyte cells are conveyed from the conduit 19 and settle further down in the bag, leaving solely plasma in the conduit 19. Thus, the buffycoat layer that has settled in the conduit is prevented from accompanying fluid to the side container 2. This enables, in turn, the conduit 19 to be given a relatively large cross-sectional area, which enables the sluggish cell fraction to be pressed from the bag more readily. If the conduit 10 of the Figure 1 embodiment is given a large cross-sectional area, a large quantity of buffycoat will accompany and contaminate the cell concentrate. Because the conduit 19 is filled with plasma, the buffycoat layer moving upwards in the bag is unable to enter said conduit. The conduit 19 may be provided with a laterally directed opening 20, in order to further reduce the risk of buffycoat being pressed into the conduit 19.

Figure 3 is a longitudinal section view of one embodiment of a connector 8 provided in one edge of the blood bag. The remaining bag connectors are also gathered in the same edge, and include a connector 7 for a hose which leads to a side container for collecting plasma, and a connector 9 for the connection of a blood drainage hose, and two further connectors, not clearly shown in the Figure. One or both of these connectors are used in subsequent processing of the residual contents of the bag.

The connector 8 includes a sleeve 11 whose one end is welded to the bag and whose other end is connected to a hose 4 which leads to a side container for collecting erythrocyte cells. Seated within the sleeve is an axially moveable hollow rod 12. The rod 12 can be forced down into the sleeve, so as to form a sleeve extension in the bag, such that the connecting conduit leading to the side container 2 will terminate at a desired distance from the bottom of the bag. The upper end 13 of the rod 12 may be rounded, so as to enable the sleeve to be readily pressed manually from the bag, while the bottom end may be pointed, as at 14, for the purpose of puncturing a sealing membrane 15 located in the sleeve attachment in the bag. The rod is also preferably provided with shoulders or fins 16, which prevent the rod from being pressed free of the sleeve 11. The shoulders may also function to effect a seal against the wall of the lead-through or bushing in the top of the bag. Figure 4 illustrates the connector shown in Figure 5, subsequent to having pressed the rod 12 out of the sleeve. In this embodiment, the length of the rod 12 is adapted so that the rod orifice is located in the layer of red blood cells present in the lower part of the bag subsequent to centrifugation, i.e. the rod has an insertable length which corresponds at least to half the length of the bag and which preferably corresponds to the full length of said bag. Alternatively, the rod can be given a length corresponding to the short conduit 19 of the Figure 2 embodiment.

According to one embodiment of the invention, the sleeve 11 consists of a relatively soft hose which can be compressed elastically in its axial direction. This enables the rod to be moved axially in the sleeve, by repeated axial compression of the hose. When the hose is compressed along the rod, the internal diameter of the hose is widened and the rod will move stepwise axially in the hose.

Figures 5 and 6 are longitudinal sectional views of another embodiment of a connection comprising an axially displaceable hollow rod which is pressed into the blood bag. Figure 5 illustrates the connection prior to pressing the rod into the bag, whereas Figure 6 illustrates the connection subsequent to having pressed-in said rod. Distinct from the embodiment illustrated in Figures 4 and 5, the hollow rod 12 of this embodiment is fixedly connected to a protective sleeve 17 and the upper end of the rod is connected to the hose 4, wherein the protective sleeve can also be used as a connecting element. The protective sleeve is so arranged that said sleeve will pleat or shirr around the rod 12 when said rod is pressed-in through a sealing lead-through or bushing 18 provided in the top of the bag. Pleating of the sleeve is either achieved by providing the sleeve with fold-lines, or by providing said sleeve with a wall of such thinness that the sleeve is pleated spontaneously when compressed.

The inventive blood bag can be used in the following way:

Blood is drained into the blood bag, for instance directly from a blood donor, through a blood drainage hose leading to a connector 9 at the top of the bag. Normally, about 450 ml of blood is drained into the bag, said blood being admixed with about 63 ml anticoagulant (e.g. CPD-solution), which may have been introduced into the bag prior to its delivery or which is introduced during the blood draining process.

When draining of the blood is completed, the blood draining hose is sealed-off, for instance with the aid of a hose clamp, and the blood bag with the side containers hanging therefrom, is placed in the centrifuge. Centrifugation of the blood causes the blood to separate into the components plasma, buffycoat and red blood cells, which settle in layers in the aforesaid order, from the top to the bottom of the bag. The different component layers also have certain divisions within themselves. Thus, the older red blood cells settle furthest down in the blood bag (= highest specific weight), with the blood-cell population of normal age located thereabove. The upper layer in the cell sediment contains the youngest red blood cells, so-called neocytes, which have particular therapeutic value. Due to their lower specific weight, the white blood cells layer on top of the uppermost red blood-cell sediment, or in the boundary layer. The blood plates, which have a still further lower specific weight, stratify on the layer of white blood cells. The uppermost layer in the blood bag contains the plasma, which is either essentially free of cells or rich in blood platelets, depending on the centrifuging technique applied and also depending on the g-factor and centrifugation time, i.e. G-seconds.

These circumstances can be utilized to produce an erythrocyte cell concentrate of particularly high quality when using the novel blood bag, in that the erythrocyte cells which settle furthest down in the bottom of the bag remain in the bag together with the buffycoat fraction when pressing the plasma and the erythrocyte cells from the bag. In the case of the Figure 1 embodiment, this can be effected by adapting the length of the conduit 10 (the rod 12), and in the Figure 2 embodiment by pressing to the desired level. In this latter case, the blood fractions are pressed from the bag with the aid of a squeezing device or press which first squeezes the bottom of the bag 1 and then squeezes the bag successively to the top thereof. The erythrocyte cells which settle furthest down in the bag are the last to reach the mouth of the conduit 19. That portion which remains will contain the major part of the oldest and therapeutically least valuable erythrocytes. The erythrocyte cell concentrate isolated in the side container 2 will thus obtain an improved therapeutic quality, owing to a higher proportion of young cells present.

Thus, subsequent to centrifuging the blood, the plasma located at the highest level in the bag will be pressed out through the connection 7 and the hose 5, into the plasma side-container 3, whereafter the hose 5 can be sealed-off and cut by means of a tube sealer for instance. The red blood cells are pressed from the bag through the conduit 19 or the conduit 10 (with the conduit 10 or the rod 12 at a selected distance from the bottom of the bag) and the hose 4 and into the side container 2 for erythrocyte cells. Remaining in the blood bag are the buffycoat fraction and minor quantities of plasma and erythrocyte cells. This remaining fraction can then be processed in a further purifying stage.

It will be understood that the novel blood bag illustrated in Figure 1 can be used upside down in the centrifuge, wherein the edge provided with the connectors 7 and 8 will be distal from the rotational center. In this case, the plasma fraction is taken out via the connector 8 (and the conduit 10/the rod 12) and the red blood cells through the connector 7. The possibility of allowing the oldest red blood cells to remain in the bag is lost in this case, however.

The blood components can be pressed from the bag with the aid, for instance, of a device of the kind described in Swedish Patent Specification No. 7902760-3. When using this device, the blood bag is placed between a fixed support plate and a moveable press plate, which is moved successively towards the support plate while monitoring the component layers with photocells. In order to ensure that the rod 12 (the conduit 10) located in the blood bag will not limit the extent to which the bag can be compressed, at least one of the plates may be provided with a pliable or yieldable layer of, e.g. rubber or foam plastic of suitable rigidity, so as to enable the rod to be pressed into said pliable layer when the extent to which the plates are brought together is greater than the dimension of the rod.

The blood components can be pressed from the bag illustrated in Figure 2 in essentially the same manner. In this case, however, it is necessary to remove the plasma fraction first, so that the buffycoat fraction will be moved to the top of the bag, above the mouth of the conduit 19, before opening the hose 4 leading to the side container 2.

## Claims

1. A blood bag for use in separating blood into respective components, said bag having at least two connections from which hoses extend to side containers and which is intended for the sterile transfer of separated blood components, **characterized** in that the connections are disposed in one edge of the bag; and in that one connection (7) is connected to the blood bag at said edge as a direct or an openable connection; and in that the other connection (8) is connected to the interior of the blood bag, at a given distance from said edge, as a direct or an openable connection.

2. A blood bag according to Claim 1, **characterized** in that the second connection includes a conduit (10, 19) which extends into the blood bag.

3. A blood bag according to Claim 1, **characterized** in that the second connection includes a sleeve (11) which is affixed to said bag edge and which surrounds a hollow, axially displaceable rod (12) which is intended to move axially in the sleeve such as to extend said sleeve in the blood bag, in response to the application of pressure on the outside of said sleeve.

4. A blood bag according to Claim 3, **characterized** in that the sleeve (11) consists of a relatively soft hose which is elastically compressible in the axial direction and in which the rod is able to move in response to repeated axial compression of the hose.

5. A blood bag according to Claim 1, **characterized** in that the second connection (8) includes a hollow rod (12) which is intended to be moved axially into the bag through a bushing (18) which seals around said rod, and further includes a protective sleeve (17) which surrounds the rod and which is intended to pleat around the rod when said rod is pushed into the bag.

6. A blood bag according to Claim 2, **characterized** in that the conduit (19) has a length such that the orifice (20) of said conduit will be located in the plasma layer obtained subsequent to centrifuging a blood unit.

## Patentansprüche

1. Blutsack zum Gebrauch beim Trennen von Blut in die jeweiligen Komponenten, wobei dieser Sack wenigstens zwei Verbindungen aufweist, von denen sich Schläuche zu Seitenbehältern erstrecken, und der zum sterilen Transfer von getrennten Blutkomponenten vorgesehen ist, dadurch gekennzeichnet, daß die Verbindungen an einer Kante des Sacks angeordnet sind; und daß eine Verbindung (7) mit dem Blutsack an dieser Kante als eine direkte oder eine zu öffnende Verbindung verbunden ist; und daß die andere Verbindung (8) in einem vorbestimmten Abstand von dieser Kante als direkte oder zu öffnende Verbindung mit dem Inneren des Blutsacks verbunden ist.

2. Blutsack nach Anspruch 1, dadurch gekennzeichnet, daß die zweite Verbindung eine Röhre (10,19) umfaßt, die sich in den Blutsack erstreckt.

3. Blutsack nach Anspruch 1, dadurch gekennzeichnet, daß die zweite Verbindung eine Hülse (11) umfaßt, die mit dieser Sackkante verbunden ist und die eine hohle, axial verschiebbare Stange (12) umhüllt, die dazu vorgesehen ist, sich axial in der Hülse so zu bewegen, daß diese Hülse in den Blutsack verlängert wird, in Reaktion auf die Ausübung von Druck auf die Außenseite dieser Hülse.

4. Blutsack nach Anspruch 3, dadurch gekennzeichnet, daß die Hülse (11) aus einem verhältnismäßig weichen Schlauch besteht, der in axialer Richtung elastisch zusammendrückbar ist und in dem die Stange in Reaktion auf wiederholtes axiales Zusammendrücken des Schlauchs bewegbar ist.

5. Blutsack nach Anspruch 1, dadurch gekennzeichnet, daß die zweite Verbindung (8) eine Hohlstange (12) umfaßt, die zum axialen Bewegen in den Sack durch eine Durchführung (18), die diese Stange abdichtend umschließt, vorgesehen ist, und daß die zweite Verbindung (8) weiter eine Schützhülse (17) umfaßt, die die Stange umgibt und die dazu vorgesehen ist, sich um die Stange zu falten, wenn diese Stange in den Sack gedrückt wird.

6. Blutsack nach Anspruch 2, dadurch gekennzeichnet, daß die Röhre (19) eine solche Länge aufweist, daß die Mündung (20) dieser Röhre sich in der Plasmaschicht befindet, die nach dem Zentrifugieren einer Bluteinheit erhalten wird.

## Revendications

1. Sac à sang servant dans la séparation du sang en ses composants respectifs, ledit sac ayant au moins deux connexions à partir desquelles des tuyaux s'étendent vers des récipients latéraux et qui est destiné au transfert stérile des composants sanguins séparés, caractérisé en ce que les connexions sont disposées dans un bord du sac; en ce qu'une connexion (7) est connectée au sac à sang audit bord sous la forme d'une connexion directe ou d'une connexion ouvrable; et en ce que l'autre connexion (8) est connectée à l'intérieur du sac à sang, à une distance donnée depuis ledit bord, sous la forme d'une connexion directe ou d'une connexion ouvrable.

2. Sac à sang selon la revendication 1, caractérisé en ce que la seconde connexion comprend un conduit (10̸,19) qui s'étend jusque dans le sac à sang.

3. Sac à sang selon la revendication 1, caractérisé en ce que la seconde connexion comprend un manchon (11) qui est fixé audit bord du sac et qui entoure une tige (12) creuse déplacable axialement qui est destinée à se déplacer axialement dans le manchon de facon à prolonger ledit manchon dans le sac à sang en réponse à l'application d'une pression depuis l'extérieur dudit manchon.

4. Sac à sang selon la revendication 3, caractérisé en ce que le manchon (11) est formé d'un tuyau qui est relativement souple, qui est compressible élastiquement dans la direction axiale et dans leauel la tige peut se déplacer en réponse à une compression axiale répétée du tuyau.

5. Sac à sang selon la revendication 1, caractérisé en ce que la seconde connexion (8) comprend une tige creuse (12) qui est destinée à être déplacée axialement jusque dans le sac au travers d'une bague (18) qui assure l'étanchéité autour de ladite tige, et en ce qu'elle comprend en outre un manchon protecteur (17) qui entoure ladite tige et qui est destiné à être plissé autour de la tige lorsque la tige est poussée dans le sac.

6. Sac à sang selon la revendication 2, caractérisé en ce que le conduit (19) a une longueur telle que l'orifice (20̸) dudit conduit sera positionné dans la couche de plasma obtenue à la suite de la centrifugation d'une unité sanguine.
